# EUROPEAN PATENT APPLICATION

(11) **EP 2 014 641 A2**
(43) Date of publication of application: **14.01.2009**
(21) Application number: 07109689.5
(22) Date of filing: 06.06.2007
(51) Int. Cl.: C07C 201/08, C07C 205/06

(54) **Process for preparing mixtures of diphenylmethane diisocyanates and polyphenyl polymethylene polyisocyanates**

(71) Applicant: HUNTSMAN INTERNATIONAL LLC, Salt Lake City, Utah 84108 (US)
(72) Inventor: The designation of the inventor has not yet been filed
(74) Representative: Swinnen, Anne-Marie

(57) **Abstract**

The present invention relates to a process for preparing mixtures of diphenylmethane diisocyanates and polyphenyl polymethylene polyisocyanates by sequential reaction of benzene to nitrobenzene to aniline to polyphenyl polymethylene polyamines wherein the benzene used as the starting material contains 500 to 5000 ppm w/w toluene and/or xylenes.

## Description

The present invention relates to a process for preparing mixtures of diphenylmethane diisocyanates (MDI) and polyphenylpolymethylene polyisocyanates, known as PMDI, by reaction of the corresponding mixtures of diphenylmethanediamines (MDA) and polyphenylpolymethylenepolyamines, known as PMDA, with phosgene in the presence of at least one inert organic solvent.

PMDI is an industrially important isocyanate for producing rigid polyurethane foams which are preferably used as insulation material in the building industry, as insulating foam in the refrigeration appliance industry and as sandwich panel construction material. Usually, part of the diphenylmethane-4,4'-diisocyanate, known as MMDI, present in the PMDI, is recovered by means of a suitable technological operation such as distillation or crystallization. MMDI is in turn an important constituent of polyurethane formulations for compact, microcellular and cellular polyurethanes such as adhesives, coatings, fibers, elastomers and integral foams. Likewise, various mixtures of the diisocyanate isomers in varying proportions (so-called "Mixed Isomer" products) can be prepared. Accordingly, the term "PMDI" as used herein also encompasses PMDI mixtures in which monomeric MDI, for example 4,4'-, 2,2'- and/or 2,4'-MDI, is present.

PMDI is manufactured commercially by the sequential conversion of benzene to nitrobenzene to aniline which then, by acid catalysed reaction with formaldehyde, forms the corresponding mixtures of diphenylmethanediamines and polyphenyl-polymethylenepolyamines, known as PMDA. Phosgenation of PMDA produces PMDI.

Similarly, toluene is converted to dinitrotoluene (typically the 80:20 mixture of 2,4- and 2,6-isomers although others are also well known) which is catalytically hydrogenated to produce the corresponding mixture of toluene diamine isomers (TDA) which, when phosgenated and distilled yields the other major commercial aromatic isocyanate - TDI.

The preparation of PMDI by reaction of the corresponding mixtures of diphenylmethanediamines and polyphenylpolymethylenepolyamines, known as PMDA, with phosgene is well known and widely practiced. Representative patents and the references contained therein illustrate some of the phosgenation techniques, such as design of mixing nozzles and other process equipment and close control of reaction conditions through the process, and are as follows:

US 2006/0041166 describes a process for the continuous preparation of organic isocyanates through the reaction of organic amines with phosgene in the presence of organic solvents under pressure whereby a concentrated phosgene-containing stream is mixed preferentially with an amine-containing stream in a jet mixer to create a combined jet of reacting amine-phosgene mixture.

WO 2004/080587 discloses an invention which relates to a method for producing polyisocyanates by reacting primary amines with phosgene comprising the following steps: a) mixing the amine with the phosgene, b) reacting the amine with the phosgene in a reactor inside of which the mixture stays for a period of time and, optionally, c) transferring the discharge from the reactor from step b) into a distillation column. The invention is characterized in that the reactor cited in step b) is provided in the form of a tubular reactor.

US 6576788 describes a process for preparing mixtures of diphenylmethane diisocyanates and polyphenylpolymethylene polyisocyanates having a reduced content of chlorinated byproducts and a reduced iodine color number by two-stage reaction of the corresponding mixtures of diphenylmethanediamines and polyphenyl-polymethylenepolyamines with phosgene in the presence of at least one inert organic solvent at elevated temperature; separation of the excess phosgene and solvent after the phosgenation is complete and thermal treatment of the reaction product; the mass ratios of phosgene to hydrogen chloride in the residence time apparatus of the second stage of the phosgenation are at the same time 10-30:1 in the liquid phase and 1-10:1 in the gas phase.

US 2006/025556 describes a two-stage process for the preparation of organic isocyanates by reacting primary amines with phosgene in which a) in a first stage, amine and phosgene are reacted in an adiabatically managed reaction in which the temperature of reaction is restricted to values between 100 and 220°C by actively adjusting the absolute pressure in the reactor to values between 8 and 50 bar by decompression and the temperature is held at values between 100 and 220°C until the stoichiometric conversion of phosgene has reached at least 80 % and then b) in a second stage, the reaction mixture from a) is decompressed to an absolute pressure of 1 to 15 bar and the reaction mixture is reacted further at temperatures between 90 and 240°C optionally with the introduction of heat.

Preparations of PMDA from the acid-catalysed reaction of formaldehyde in various forms with aniline is also well known and widely practiced with a wide range of process equipment configurations and reaction and work-up conditions. The continuous, discontinuous, or semi-continuous preparation of di- and polyamines of the diphenylmethane series, called PMDA is described in numerous patents and publications [see, for example JP 9406590 , DE 19804915, EP 1616890 and references therein and also H. J. Twitchett, Chem. Soc. Rev. 3(2), 209 (1974), M. V. Moore in Kirk-Othmer Encycl. Chem. Technol., 3rd ed., New York, 2, 338-348 (1978)]. The preparation of these polyamines is conventionally carried out by reaction of aniline and formaldehyde in the presence of acidic catalysts. Aqueous hydrogen chloride is conventionally employed as the acidic catalyst. According to the prior art, the acidic catalyst is neutralized by addition of a base, and thus, used up at the end of the process, and before the final working-up steps (such as, for example, removal of excess aniline by distillation). The use of solid acid catalysts is also well known [see, for example, Trends in industrial catalysis in the polyurethane industry, Applied Catalysis A : General 221, 303-335 (2001)].

The preparation of aniline from the catalysed hydrogenation of nitrobenzene is also well known and widely practiced with a wide range of process equipment configurations and reaction and work-up conditions [see, for example, *"*Aniline and derivatives", Report No.76C, 1993, SRI international issue].

Generally, the hydrogenation of nitroaromatics to the equivalent amine, e.g. nitrobenzene to aniline and dinitrotoluene to toluenediamine, usually has been carried out by effecting the hydrogenation in liquid phase over a hydrogenation/dehydrogenation catalyst such as Raney nickel. Normally in the hydrogenation process, the nitrated product is purified and removed of acidic material and alkaline material which act as catalyst poisons in the hydrogenation reaction. US 4224249 discloses such a process for this hydrogenation. Gas phase hydrogenations are also well-known.

Numerous processes have been developed for effecting nitration of aromatic hydrocarbons generally; some have focused on the synthesis of mononitroaromatics while others are focused on the production of dinitro or trinitro aromatic compositions. Nitration of aromatic compositions have typically been done by the mixed acid technique, i.e. a mixture of nitric acid and sulfuric acid, although nitration of aromatics has also been affected utilizing nitric acid alone or mixtures of nitrogen oxides and sulfuric acid. Representative patents illustrating some of the nitration techniques are as follows:

US 2362743, US 2739174 and US 3780116 disclose processes for the nitration of aromatic hydrocarbons using nitric acid as the sole nitrating medium. US 2362743 uses a two-stage nitration process to form dinitrotoluene in the first stage, toluene is nitrated with 60-75% nitric acid at temperatures about 75 - 80°C and then dinitrated with 90-100% nitric acid at the same temperature. In US 2739174 benzene, toluene, and xylene were nitrated using 70% nitric acid at temperatures of from about 110 -120°C. In this process, a liquid reaction mixture comprising water, nitric acid and nitrated hydrocarbon was withdrawn from the reactor and the nitric acid separated from the water nitrated hydrocarbon azeotrope via distillation. US 3780116 used approximately 40% nitric acid as the nitrating medium for benzene and toluene and the process involved bubbling hydrocarbon vapor through the nitric acid medium at temperatures of from about 50 - 100°C. A nitrobenzene-nitric acid mixture is withdrawn from the reactor and the mixture separated by decavitation. Nitric acid and unreacted benzene and water are removed as vapor with the benzene being separated and returned.

By and large the technique for nitrating aromatic hydrocarbons such as benzene, toluene, xylene, naphthalene, anthraquinone has been the mixed acid technique. US 2256999, US 2370558, US 2773911, US 2849497, US 3434802, US 4021498 and US 4112005 disclose variations in the mixed acid technique for producing nitroaromatic compositions and particularly, mono and dinitroaromatic compositions. The mixed acid technique is preferred in the manufacture of nitroaromatics since the concentration of the nitronium ion, which is the nitrating agent, is much lower in nitric acid alone than in the mixed acid. In the mononitration of aromatics, the aromatic hydrocarbons are contacted with a nitric acid/sulfuric acid mixture, the nitric acid concentration typically being about 20-70% by volume or more dilute than in dinitration reaction; the sulfuric acid typically used in 80-98% concentration.

US 4112005 mentioned above discloses preparing the mononitroaromatic compounds by nitrating a reactive aromatic compound in the absence of sulfuric acid until mononitration is complete, the nitration being carried out at 40-68% by weight nitric acid.

Dinitroaromatics, e.g. dinitroxylene and particularly dinitrotoluene have been typically produced by using highly concentrated nitric acid compositions or the mixed acid technique and US 2362743, US 2934571 and US 3092671 are representative. US 2362743 effects dinitration of toluene in the absence of sulfuric acid. The mononitration is carried out with 70% nitric acid, while the dinitration is carried out using 98% nitric acid at temperatures of about 70 - 80°C. High mole ratios of acid e.g. 2-5:1 moles nitric acid per mole hydrocarbon, are required. US 2934571 discloses the nitration of various aromatics such as benzene, nitrobenzene, halogen-substituted benzenes, and so forth by the mixed acid technique. In that process a mixture of fuming nitric acid and fuming sulfuric acid are reacted with the aromatic hydrocarbon at temperatures of 50 - 60°C.

Commercially, the nitration of toluene to form dinitrotoluene is done in a two-step process wherein mononitrotoluene is formed in a first stage, the water of reaction and spent acid being removed from the mononitrobenzene reaction product and then the mononitrobenzene charged to the dinitrator for subsequent nitration.

So-called "nitration grade" benzene is available at over 99.9 wt% with impurities of toluene and hydrocarbon (C6/C7) compounds [Netzer & Ghalayini in Hydrocarbon Processing, April 2002, pages 71-78], where the toluene level is under 500 ppm [Netzer & Ghalayini in Benzene Recovery from Refinery Sources by co-production of olefins, presented at the National Petrochemical & Refiners Association annual meeting, March 23-25, 2003, San Antonio, Texas]. A high purity "nitration grade" toluene is also available.

Conventionally pure "nitration grade" benzene is used for the sequential manufacture of nitrobenzene, aniline, PMDA and, ultimately, PMDI and, similarly, use of pure "nitration grade" toluene for the sequential manufacture of dinitrotoluene, toluene diamine and, ultimately, TDI. This way the technological requirements for impurity removal at various stages of the sequential production chain are reduced and, hence, production operational and capital costs and waste disposal costs. However, this approach of using high purity starting materials must be considered against the considerable costs of obtaining the starting materials (benzene and toluene respectively) in such a pure form. See, for example, US 5004851 and references therein for descriptions of benzene purification processes.

It has been reported (US 4185036) that mixed nitroaromatic compositions can be hydrogenated under appropriate conditions to form aromatic amines with less by-product tars at higher rates and higher yields than are achieved with prior art hydrogenation processes. More particularly, the patent discloses that a mixture of aromatic compounds can be selectively hydrogenated to the amine, the mixture containing at least 25% of a mononitro-non-aminoaromatic compound and at least 25% of a dinitro or a mononitro-amino compound. Examples of reactants which are suited for the selective cohydrogenation include o-nitrotoluene, o-nitroaniline, mononitrotoluene, dinitrobenzene, dinitrotoluene and other nitro aromatics.

US 4935557 discloses the manufacture of a mixture comprising a mononitro aromatic compound and a dinitro aromatic compound, optionally including other nitro aromatics, which can be selectively cohydrogenated to form the corresponding aromatic amine. In a preferred embodiment, the nitro aromatic composition comprises a mixture of mononitrobenzene and dinitrotoluene. The process involves the reaction of a feed mixture comprising benzene and toluene with nitric acid under conditions suited for nitration. Typically, the nitric acid concentration is from 88 to 95% by weight at the steady state and the reaction temperature is from 40 to 70°C. The reaction time is sufficient to effect mononitration of the benzene but insufficient for effecting substantial dinitration of the benzene in the feed mixture. Characteristics of this process are the ability to utilize refinery streams comprising benzene and toluene, optionally with small amounts of xylene, without prior separation to form a suitable feedstock for nitration; an ability to nitrate selectively a feed mixture to form a nitroaromatic mixture consisting primarily of mononitrobenzene and dinitrotoluene as the nitrated benzene and toluene products; an ability to form selectively mononitrobenzene and dinitrotoluene in combination with each other for further hydrogenation without a plurality of separation stages involving the separation of unstable nitroaromatic compositions and an ability to reduce the amount of process steps necessary to produce aromatic amine intermediates without numerous separation stages prior to the generation of such aromatic amine intermediates.

However, a major disadvantage of the coproduction of nitrobenzene and dinitrotoluene is the coproduction of significant amounts of dinitrobenzene and nitrotoluene (see Tables in US 4935557). Subsequent cohydrogenation of the nitrobenzene - dinitrotoluene mixture to produce aniline and toluene diamine also forms unwanted diaminobenzene and toluidine. Subsequent separation of the aniline and toluene diamine for the ultimate production of PMDI and TDI by means of the additional conventional production methods, produces diaminobenzene and toluidine waste which is unsatisfactory on economic grounds. The alternative of allowing the significant amounts of unwanted diaminobenzene and toluidine to remain either with the aniline or TDA or distributed between them would ultimately result in formation of PMDI and/or TDI with undesired compositions, due to the presence of significant amounts of impurity compounds.

Thus, there still exists a need for a process for the production of PMDI which can advantageously utilise an economically attractive less-than-pure benzene composition as the starting point of the production chain [benzene - nitrobenzene - aniline - PMDA - PMDI] which does not simultaneously produce either extra waste streams or undesired final product compositions. Surprisingly, it has been found that benzene containing 500 to 5000 ppm, preferably 500 to 1000 ppm w/w of toluene satisfies exactly these requirements.

The present invention accordingly provides a process for the production of PMDI by sequential reaction of benzene to nitrobenzene to aniline to PMDA to PMDI, wherein the benzene used as the starting material contains 500 to 5000 ppm, preferably 500 to 1000 ppm w/w of toluene. Similarly, the present invention also applies to benzene containing these same levels (500 to 5000 ppm, preferably 500 to 1000 ppm w/w) of xylenes and also to apply to benzene containg varying combinations of toluene and xylenes where the total level of these impurities is in the 500 to 5000 ppm w/w range.

Surprisingly, we have found that benzene containing low levels of alkyl-substituted aromatics, notably toluene, can be used in the conventional mixed acid nitration process without recourse to any changes in the production process equipment, process reaction conditions or process control mechanisms.

Typical process conditions for the mixed acid nitration of benzene can be found, for example, in Nitration - Methods and Mechanisms, Olah, Malhotra and Narang, VCH Publishers Inc, New York; Nitration - Recent Laboratory and Industrial Developments, ed. Albright, Carr and Schmitt, ACS, Washington, D.C. and references therein and in descriptions of the Noram Engineering and Constructors Ltd. nitrobenzene process (e.g. US 4994242).

The crude nitrobenzene containing low levels of nitrotoluene isomers produced in the mixed acid nitration process is worked-up with conventional processes (separation and re-concentration of sulphuric acid, washing of the aromatic product stream to remove inorganics and distillation of benzene for subsequent recycle).

Surprisingly, we have found that the nitrobenzene containing low levels of nitrotoluene isomers can be used in the conventional process for making aniline without recourse to any changes in the production process equipment, process reaction conditions or process control mechanisms. Thus, the nitrobenzene containing low levels of nitrotoluene isomers can be converted to aniline containing low levels of toluidene isomers by hydrogenation over metal or supported-metal catalysts. Typical process conditions for aniline production process can be found in, for example, GB 982902 and GB 982903.

Surprisingly, we have found that the aniline containing low levels of toluidene isomers can be used in the conventional process for making diphenylmethanediamines and polyphenylpolymethylenepolyamines (known as PMDA) without recourse to any changes in the production process equipment, process reaction conditions or process control mechanisms. Thus, the aniline containing low levels of toluidene isomers can be reacted with formaldehyde in the presence of acid catalysts to make diphenylmethanediamines and polyphenylpolymethylenepolyamines where the toluidene isomers become incorporated into mixed polyamine molecules. "Mixed" in the context used herein indicates molecules containing one or more aniline moieties and, in most cases, one toluidene isomer linked together by methylene groups derived from the formaldehyde [the presence of molecules containing more than one toluidene isomer is theoretically possible but such compounds will only be present in extremely low levels, if at all].

Such PMDAs are advantageously obtained by condensation of aniline and formaldehyde in a molar ratio of 6-1.6:1, preferably 4-1.9:1, and a molar ratio of aniline to acid catalysts of 1:0.98-0.01, preferably 1:0.8-0.1. The formaldehyde can be used in any physical form (solid, liquid or gas) and is preferably used in the form of an aqueous solution, e.g. as a commercial 30-55 % strength by mass solution. Acid catalysts which have been found to be useful are proton donors such as acid ion exchange resins or strong organic and preferably inorganic acids. For the purposes of the present invention, strong acids are those having a pKa of less than 1.5; in the case of polybasic acids, this value is that for the first hydrogen dissociation. Examples which may be mentioned are hydrochloric acid, sulfuric acid, phosphoric acid, fluorosulfonic acid and oxalic acid. Hydrogen chloride in gaseous form can also be used. Preference is given to using aqueous hydrochloric acid in concentrations of from about 25 to 33% by mass. Suitable processes for preparing PMDA are described, for example, in CA 700026, DE 2227110 (equivalent to US 4025557), DE 2238920 (equivalent to US 3996283), DE 2426116 (equivalent to GB 1450632), DE 1242623 (equivalent to US 3478099), GB 1064559 and DE 3225125.

Processes based on condensation of the aniline containing low levels of toluidene isomers with formaldehyde under neutral or basic conditions, with subsequent optional separation of water and, additionally, optional further drying of the condensate can also be used. The so-called neutral condensate, containing N,N'-methylene dianiline (aminal) and possibly other anilinoacetals and the methyl-substituted analogues is then subsequently converted to secondary amines and the final primary amine mixture PMDA by using acidic species. These can be the same acid catalysts as described above but other heterogeneous solid acid catalysts described in the prior art could also be used (see, for example, Trends in industrial catalysis in the polyurethane industry, Applied Catalysis A : General 221, 303-335 (2001) and US 3362979, US 4039580 and US 4039581).

Surprisingly, we have found that this unconventional PMDA [diphenylmethanediamines and polyphenylpolymethylenepolyamines with low levels of toluidene isomers incorporated into mixed polyamine molecules] can be used in the conventional process for making PMDI without recourse to any changes in the production process equipment, process reaction conditions or process control mechanisms.

The phosgenation of primary amines in a mixing reactor as first stage of the phosgenation has been described a number of times. Thus, for example, US 3544611 and EP 150435 report the phosgenation in a pressure mixing circuit. Furthermore, EP 291819 discloses carrying out this reaction in a reaction pump. Many different designs of static mixers have been described, for example: annular slot nozzle (FR 2325637, DE 1792660), ring-eye nozzle (DE 3744001), flat jet nozzle (EP 65727), fan jet nozzle (DE 2950216), angle-jet chamber nozzle (DD 300168), three-fluid nozzle (DD 132340), coaxial jet mixer nozzle with protruding centerbody (US 2004008572). The temperature in the first stage of the phosgenation is usually from 40 to 150°C, preferably from 60 to 130°C, particularly preferably from 90 to 120°C. By allowing the exothermic reactions taking place to increase the temperature of the mixture to above approximately 80°C, solidification of carbamoyl chlorides can be prevented. Careful design of the mixing device minimises urea by-product formation by minimising contacting of incoming amine with reaction products, such that formation of insoluble "poly-ureas" is avoided. Formation of some urea functional groups is not problematic since these will be simultaneously present in compounds also containing polyisocyanates and, thus, such "mixed functionality" compounds will be soluble in the mixture of normal polyisocyanates.

In a subsequent stage the corresponding carbamoyl chlorides and amine hydrochlorides formed in the first stage of the phosgenation can be run through many types of residence time apparatus in which the amine hydrochlorides are phosgenated to form the corresponding carbamoyl chlorides and the carbamoyl chlorides are dissociated into the corresponding isocyanates and hydrogen chloride. For example, the mixture from a previous stage of the phosgenation can be fed to a series of stirred tank reactors, tubular or column reactors or thin film devices (such as in WO 2004/031132) or combinations of different types of reactors. Batch, continuous, semi-continuous processes and combinations of these, operating at atmospheric pressure or above, are all known in the art.

The PMDI mixtures prepared by the process of the present invention usually have a diphenylmethane diisocyanate isomer content of from 30 to 90% by weight, preferably from 30 to 70% by weight, an NCO content of from 29 to 33% by weight, preferably from 30 to 32% by weight, based on the weight of crude MDI, and a viscosity, determined at 25°C in accordance with DIN 51550, of not more than 2500 mPa.s, preferably from 40 to 2000 mPa.s.

Crude PMDI's having such isomer and homologue compositions can be prepared by phosgenation of unconventional PMDA's having corresponding product compositions in the presence of at least one solvent.

The other starting component for preparing crude PMDI is phosgene. The phosgene can be used as liquid or gas, diluted in solvents or with other gases which are inert under the reaction conditions, e.g. monochlorobenzene, ortho dichlorobenzene, nitrogen, carbon monoxide, etc. The molar ratio of unconventional PMDA to phosgene is advantageously selected such that from 1 to 10 mole, preferably from 1.2 to 4 mole, of phosgene are present in the reaction mixture per mole of NH₂ groups. The phosgene can all be fed into the first stage of the phosgenation or part of it can also be added to the residence time apparatus of the subsequent stage of the phosgenation.

Suitable solvents are compounds in which the unconventional PMDA and the phosgene are at least partially soluble. Solvents which have been found to be useful are chlorinated, aromatic hydrocarbons, for example monochlorobenzene, dichlorobenzenes such as o-dichlorobenzene and p-dichlorobenzene, trichlorobenzenes, the corresponding toluenes and xylenes, chloroethylbenzene, monochlorobiphenyl, alpha- or beta-naphthyl chloride and dialkyl phthalates such as diethyl isophthalate. Isocyanate compounds or mixtures other than MDI's or, preferably, crude or purified PMDI or other MDI material can also be used to replace some or all of the non-isocyanate solvent after the unconventional PMDA has been initially reacted with the phosgene. Excess phosgene can also be used to take the role of the solvent. Particular preference is given to using monochlorobenzene (MCB), dichlorobenzenes or mixtures of these chlorobenzenes as inert organic solvents. The solvents can be used individually or as mixtures. It is advantageous to use a solvent which has a boiling point lower than that of the MDI isomers so that the solvent can easily be separated from the crude PMDI by distillation. The amount of solvent is advantageously selected such that the reaction mixture has an isocyanate content of from 2 to 40% by mass, preferably from 5 to 20% by mass, based on the total weight of the reaction mixture.

The unconventional PMDA can be employed as such or as a solution in organic solvents. However, particular preference is given to using unconventional PMDA solutions having an amine content of from 2 to 45% by mass, preferably from 25 to 44% by mass, based on the total weight of the amine solution.

Dependent upon the exact design of the phosgenation reaction section and the conditions of temperature and pressure selected, varying proportions of phosgene, hydrogen chloride, solvent and other components of the complex reaction mixture will be partitioned between vapor, solution and solids phases. The vapor phase may be largely or partially separated from or may be kept in direct contact with the solution and solids during different stages of the phosgenation.

Subsequent to the phosgenation stages, the reaction mixture is worked-up such that remaining excess phosgene and hydrogen chloride and the solvent are preferably separated from the reaction product. The work-up procedure also includes a thermal treatment step (the so-called "dechlorination") which is likewise well known in the art. The crude PMDI may then be further treated to produce diisocyanate and polymeric MDI products. The minor levels of methyl-substituted molecules present in the PMDI resulting from the use of benzene containing low levels of toluene are not deleterious to conventional final applications of PMDI.

An additional object of the present invention is the surprising discovery that the absorption spectra of samples of less-than-pure benzene vary sufficiently even when there are only relatively small concentration differences and impurities are at relatively low levels, such that one can determine the impurity concentrations on the basis of measuring the spectrum, preferably in the NIR region, of the less-than-pure benzene, with the aid of a chemometric calibration model.

Hence the present invention also provides for process control of the initial nitration process by analysis of the less-than-pure benzene stream since it is a requirement that the level of impurities does not significantly exceed the specified levels otherwise problems arise within different stages of the production chain and, ultimately, the final product may not meet customer requirements and, hence, be commercially unacceptable. Quality monitoring has been carried out conventionally by taking samples of the benzene and by, for example, subsequent manual chromatographic analysis, preferably gas chromatography (GC), of these samples. In the case of sample production streams, it is necessary to take occupational safety and environmental protection conditions into account, in order to avoid risks involved in the handling of benzene samples. Furthermore, the number of samples that can be realistically taken is limited due to the associated labour cost, and information about the composition of the sample is not available until after a significant delay. Thus, in controlling the process, this manual method has significant disadvantages.

With manual controls and sampling, it is conceivable that the benzene quality being used may have a relatively large difference in isomer content from the setpoint composition, particularly over relatively long periods of time. This can result in a reduction of the product quality or the production of waste.

Process chromatography or online spectroscopic methods, especially near-infrared (NIR), medium infrared (mid-IR) or Raman spectroscopy can all be used for analyzing or assessing the quality of the benzene raw material. The analytical method of near-infrared (NIR) spectroscopy is a widespread technique, which is used both in the laboratory and in online operation. The combination of NIR spectroscopy with chemometric evaluation methods for special measurement tasks is likewise known per se from the prior art as described in, for example, DE 2139269, WO 97/41420, WO 98/29787, WO 99/31485, JP 11350368, WO 2002/0834, JP 2000146835, JP 2000298512, WO 2002/04394, WO 2002/12969, WO 95/31709, US 5707870, US 5712481 and WO 2000/68664. The advantages of combining optical fibers and an NIR spectrometer, compared with using medium-infrared spectroscopy, are known from Khetty (see "In-line monitoring of polymeric processes" Antec '92, 2674-2676).

In order to use NIR spectroscopy in the field of quantitative determinations, the analytical method is frequently used in combination with chemometric evaluation methods. For example, it is customary to use the partial least-squares (PLS) method in this case, as can be found and described, for example, by Raphael Vieira in "In-line and In Situ Monitoring of Semi-Batch Emulsion Copolymerizations Using Near-Infrared Spectroscopy" J. Applied Polymer Science, Vol. 84, 2670-2682 (2002), or by T. Rohe in "Near Infrared (NIR) spectroscopy for in-line monitoring of polymer extrusion processes" Talanta 50 (1999) 283-290, or by C. Miller in "Chemometrics for on-line spectroscopy applications-theory and practice", J. Chemometrics 2000; 14:513-528 and in"Multivariate Analysis of Near-Infrared Spectra Using G-Programming Language" J. Chem. Inf. Comput. Sci. 2000, 40, 1093-1100.

The use of NIR techniques for special measurement tasks is furthermore known and described in, for example, WO 00/02035, US 5717209, US 6228650, WO 99/31485, US 6339222, WO 00/68664 and DE 10005130. A review of the use of multivariate chemometric calibration models in analytical chemistry is also provided by "Multivariate Calibration", Jörg-Peter Conzen, 2001, ISBN 3-929431-13-0. In the prior art, however, such spectroscopic methods are not used for the on-line monitoring of less-than-pure benzene for production of nitrobenzene as part of the production chain to make PMDI.

## Claims

1. Process comprising the step of nitration of benzene to nitrobenzene, **characterised in that** the benzene used as the starting material contains 500 to 5000 ppm w/w alkyl-substituted aromatics.

2. Process according to claim 1 wherein the benzene contains 500 to 1000 ppm w/w alkyl-substituted aromatics.

3. Process according to claim 1 or 2 wherein the alkyl-substituted aromatics are toluene and/or xylenes.

4. Process according to any one of the preceding claims wherein the nitrobenzene is subsequently converted to aniline.

5. Process according to claim 4 wherein the aniline is subsequently converted into diphenylmethanediamines and polyphenyl polymethylene polyamines.

6. Process according to claim 5 wherein the polyphenyl polymethylene polyamine is subsequently converted into diphenylmethane diisocyanates and polyphenyl polymethylene polyisocyanate.

7. A method to monitor the quality of the benzene starting material as defined in any one of claims 1 to 3 comprising :
(1) recording a spectrum of the less-than-pure benzene, and
(2) evaluating the spectrum by a chemometric calibration model, thereby determining the actual impurity concentrations.
